# EUROPEAN PATENT APPLICATION

(11) **EP 4 445 867 A1**
(43) Date of publication of application: **16.10.2024**
(21) Application number: 22904029.0
(22) Date of filing: 24.11.2022
(51) Int. Cl.: A61C 17/02

(54) **ORAL CAVITY CLEANING DEVICE**

(30) Priority: 07.12.2021 JP 2021198432
(71) Applicant: Panasonic Intellectual Property Management Co., Ltd., Kadoma-shi, Osaka 571-0057 (JP)
(72) Inventor: SHINODA, Hiroki, Kadoma-shi, Osaka 571-0057 (JP); TSUTSUI, Mami, Kadoma-shi, Osaka 571-0057 (JP); SANEMATSU, Wataru, Kadoma-shi, Osaka 571-0057 (JP)
(74) Representative: Grünecker Patent- und Rechtsanwälte PartG mbB
(86) International application number: PCT/JP2022/043277
(87) International publication number: WO 2023/106098

(57) **Abstract**

The present disclosure provides an oral cavity washing device capable of changing a liquid flow of a washing liquid without increasing the number of parts. The oral cavity washing device according to the present disclosure includes a device body including a pump that discharges a washing liquid, and nozzle that is provided in the device body and discharges the washing liquid discharged by the pump to an outside, wherein nozzle includes narrow portion in such a manner that the sectional area thereof is set to be smaller than that of another portion of nozzle, and that the sectional area of narrow portion is variable.

## Description

### TECHNICAL FIELD

The present disclosure relates to an oral cavity washing device.

### BACKGROUND ART

Conventionally, as an oral cavity washing device, there has been known an oral cavity washing device including a device body including a pump that discharges a washing liquid and a nozzle that is provided in the device body and discharges the washing liquid discharged by the pump to the outside (see PTL 1). In this oral cavity washing device, for example, water or a washing liquid in which a medical agent is mixed with water is discharged from the nozzle toward an oral cavity, thereby removing food residues and dental plaques in the oral cavity.

### Citation List

### Patent Literature

PTL 1: Unexamined Japanese Patent Publication No. 2018-79251

### SUMMARY OF THE INVENTION

### Technical problem

In the oral cavity washing device disclosed in PTL 1, a narrow portion having an inner diameter set smaller than inner diameters of other portions may be provided in a nozzle. By providing the narrow portion in the nozzle, it is possible to change the liquid flow caused by, for example, the discharge amount, the discharge load, the flow speed, and the like of the washing liquid discharged from the nozzle, and to improve the performance of removing food residues and dental plaques. However, to change the liquid flow of the washing liquid, it is necessary to use a plurality of nozzles provided with narrow portions having different inner diameters, and the number of parts increases.

The present disclosure has been made in view of such problems of the conventional technique. An object of the present disclosure is to provide an oral cavity washing device capable of changing a liquid flow of a washing liquid without increasing the number of parts.

### Solution to problem

An oral cavity washing device according to an aspect of the present disclosure includes a device body including a pump that discharges a washing liquid, and a nozzle that is provided in the device body and discharges the washing liquid discharged by the pump to an outside, wherein the nozzle includes a narrow portion in such a manner that the sectional area thereof is set to be smaller than that of another portion of the nozzle, and that the sectional area of the narrow portion is variable.

### Advantageous effect of invention

According to the present disclosure, it is possible to provide an oral cavity washing device capable of changing a liquid flow of a washing liquid without increasing the number of parts.

### BRIEF DESCRIPTION OF THE DRAWINGS

Fig. 1 is a schematic view of an oral cavity washing device according to a first exemplary embodiment.
Fig. 2 is an enlarged sectional view of a main part illustrating an example in which a narrow portion is formed by applying an external force to a nozzle of the oral cavity washing device according to the first exemplary embodiment.
Fig. 3 is an enlarged sectional view of a main part illustrating another example in which a narrow portion is formed by applying an external force to a nozzle of the oral cavity washing device according to the first exemplary embodiment.
Fig. 4 is an enlarged perspective view of a main part of a nozzle of an oral cavity washing device according to a second exemplary embodiment.
Fig. 5 is an enlarged sectional view of the main part of the nozzle of the oral cavity washing device according to the second exemplary embodiment.
Fig. 6 is an enlarged sectional view of a main part of a nozzle of an oral cavity washing device according to a third exemplary embodiment.
Fig. 7 is a front view of Fig. 6.
Fig. 8 is an enlarged sectional view of the main part of the nozzle of the oral cavity washing device according to the third exemplary embodiment.
Fig. 9 is a front view of Fig. 8.

### DESCRIPTION OF EMBODIMENT

Hereinafter, an exemplary embodiment will be described in detail with reference to the drawings. Unnecessarily detailed description may be omitted. For example, a detailed description of already well-known matters or a redundant description of substantially the same configuration may be omitted.

The accompanying drawings and the following description are provided for those skilled in the art to fully understand the present disclosure, and are not intended to limit the subject matter described in the claims in any way.

### (First exemplary embodiment)

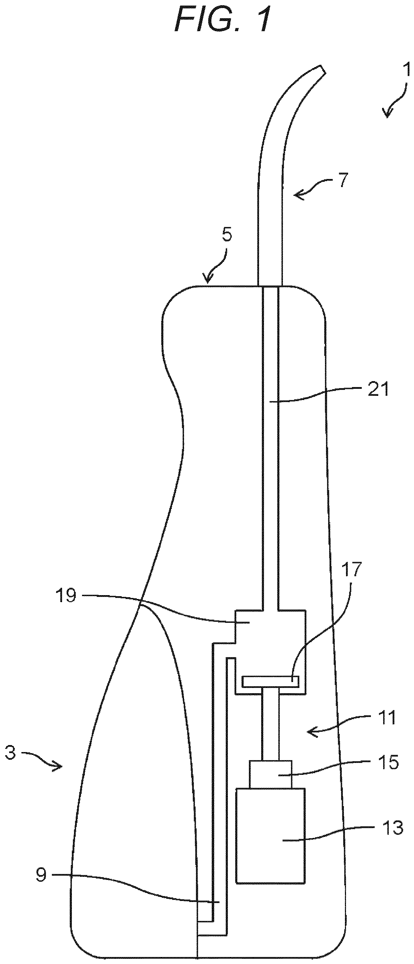

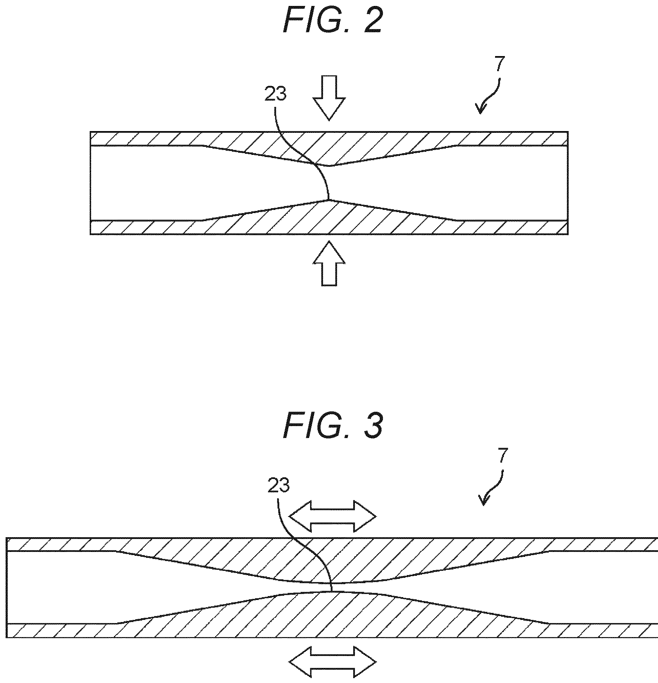

A first exemplary embodiment will be described with reference to Figs. 1 to 3. Fig. 1 is a schematic view of oral cavity washing device 1 according to the first exemplary embodiment. Fig. 2 is an enlarged sectional view of a main part illustrating an example in which narrow portion 23 is formed by applying an external force to nozzle 7 of oral cavity washing device 1 according to the first exemplary embodiment. Fig. 3 is an enlarged sectional view of a main part illustrating another example in which narrow portion 23 is formed by applying an external force to nozzle 7 of oral cavity washing device 1 according to the first exemplary embodiment.

Oral cavity washing device 1 according to the present exemplary embodiment includes tank 3, device body 5, and nozzle 7.

Tank 3 stores therein, for example, water or a washing liquid obtained by mixing a medical agent with water. Tank 3 is detachably attached to device body 5. The washing liquid is supplied to the inside of tank 3 in a state where tank 3 is detached from device body 5. Tank 3 may be provided with a supply port therein through which the washing liquid can be supplied in a state where the tank is attached to device body 5. Tank 3 supplies the washing liquid stored in tank 3 to device body 5 in a state of being attached to device body 5.

Device body 5 is formed in a housing shape larger than tank 3 so that tank 3 can be attached. Device body 5 is provided with a suction path 9 to which the washing liquid stored in tank 3 is supplied. Suction path 9 is provided with a filter (not illustrated) for removing foreign substances mixed in the washing liquid. Suction path 9 communicates with pump 11 built in device body 5, and supplies the washing liquid stored in tank 3 to pump 11 through driving of pump 11.

Pump 11 includes motor 13, cam 15, piston 17, and pump chamber 19. Motor 13 is driven by supply of electric power from a rechargeable battery (not illustrated) accommodated inside device body 5. Cam 15 converts the rotation of motor 13 into an axial operating force. Piston 17 is reciprocated along a height direction of device body 5 by the axial operating force converted by cam 15. An end of piston 17 is disposed in pump chamber 19, and the volume thereof changes due to the reciprocating motion of piston 17. Pump chamber 19 communicates with suction path 9 and discharge path 21 provided inside device body 5. A suction valve (not illustrated) is provided between pump chamber 19 and suction path 9, and a discharge valve (not illustrated) is provided between pump chamber 19 and discharge path 21.

When piston 17 moves in a direction of increasing the volume of pump chamber 19 through driving of motor 13, pump 11 causes the washing liquid in tank 3 to flow into pump chamber 19 via suction path 9. The washing liquid flowing into pump chamber 19 is discharged to nozzle 7 via discharge path 21 when piston 17 moves in a direction of reducing the volume of pump chamber 19 through driving of motor 13.

Although not illustrated, a power switch that turns on and off (ON/OFF) oral cavity washing device 1 is provided on an outer surface of device body 5. The outer surface of device body 5 is provided with an adjustment button for adjusting a liquid flow such as a discharge amount, a discharge load, and a flow speed of the washing liquid to be discharged from the nozzle.

Nozzle 7 is formed in a hollow elongated shape, and is detachable from device body 5. An end of nozzle 7 close to device body communicates with discharge path 21 in a state of being attached to device body 5. Nozzle 7 sprays the washing liquid flowing out of discharge path 21 from the tip thereof. The washing liquid sprayed from nozzle 7 removes at least one selected from the group consisting of food residues and dental plaques in the oral cavity.

As illustrated in Figs. 2 and 3, such nozzle 7 is provided inside with narrow portion 23 having a sectional area set smaller than sectional areas of other portions. In Figs. 2 and 3, as an example, narrow portion 23 having a smaller sectional area is provided inside nozzle 7 having a cylindrical shape by setting the inner diameter to be smaller than other portions. Providing narrow portion 23 in nozzle 7 makes it possible to change a liquid flow such as, a discharge amount, a discharge load, and a flow speed of the washing liquid to be discharged. Changing the liquid flow of the washing liquid makes it possible to improve the removal performance for at least one selected from the group consisting of food residues and dental plaques in the oral cavity.

To remove food residues in the oral cavity, it is necessary to increase the discharge amount and the discharge load in the liquid flow of the washing liquid. On the other hand, to remove dental plaques in the oral cavity, it is necessary to increase the flow speed because cavitation is used in the liquid flow of the washing liquid. However, for example, when the sectional area of narrow portion 23 is reduced in order to increase the flow speed of the washing liquid, the discharge amount of the washing liquid decreases. Thus, it is difficult to achieve both the removal of food residues and the removal of dental plaques.

To achieve both the removal of food residues and the removal of dental plaques, a plurality of nozzles 7 having different sectional areas of narrow portion 23 can be replaced and used each time, but the number of parts increases. Thus, in oral cavity washing device 1, the sectional area of narrow portion 23 can be varied in one nozzle 7.

Nozzle 7 is made of a material in which the shape thereof changes when an external force is applied, such as a plastically deformable resin, a plastically deformable metal, an ultraviolet curable resin, or a shape memory alloy. For example, as illustrated in Fig. 2, narrow portion 23 is formed by mechanically applying an external force from the outside to the inside of nozzle 7 using a jig or the like as indicated by the arrows to make its sectional area smaller than other portions. Alternatively, as illustrated in Fig. 3, narrow portion 23 is mechanically stretched by applying an external force toward both sides in the length direction of nozzle 7 using a jig or the like as indicated by the arrows, and is formed to have a smaller sectional area than other portions. Narrow portion 23 may be formed by applying a chemical external force such as heat or ultraviolet rays in accordance with the material used for nozzle 7 to make its sectional area smaller than other portions and not limited by a mechanical external force.

Applying an external force to nozzle 7 to form narrow portion 23 in this manner makes it possible to change the sectional area of narrow portion 23 so as to adjust the liquid flow of the washing liquid with respect to one nozzle 7. Thus, without increasing the number of parts, it is possible to adjust the liquid flow of the washing liquid so as to achieve both the removal of food residues and the removal of dental plaques, for example. When the reduced sectional area of narrow portion 23 is increased, for example, a jig may be inserted into nozzle 7, and an external force may be mechanically applied from the inside to the outside of nozzle 7. Alternatively, a chemical external force such as heat or ultraviolet rays may be applied according to the material used for nozzle 7.

As just described, oral cavity washing device 1 according to the first exemplary embodiment includes device body 5 including pump 11 that discharges a washing liquid, and nozzle 7 that is provided in device body 5 and discharges the washing liquid discharged from pump 11 to the outside. Nozzle 7 has narrow portion 23 having a sectional area set to be smaller than sectional areas of other portions of nozzle 7. the sectional area being variable.

Since the sectional area of narrow portion 23 can be changed, the liquid flow of the washing liquid to be discharged from nozzle 7 can be changed even with one nozzle 7 by changing the sectional area of narrow portion 23. Thus, it is not necessary to use a plurality of nozzles 7 provided with narrow portions 23 having different sectional areas, and the number of parts does not increase.

Thus, in such oral cavity washing device 1, the liquid flow of the washing liquid can be changed without increasing the number of parts.

The sectional area of narrow portion 23 is changed by using an external force. Thus, adjusting the external force makes it possible to adjust the sectional area of narrow portion 23 in accordance with the liquid flow of the washing liquid.

### (Second exemplary embodiment)

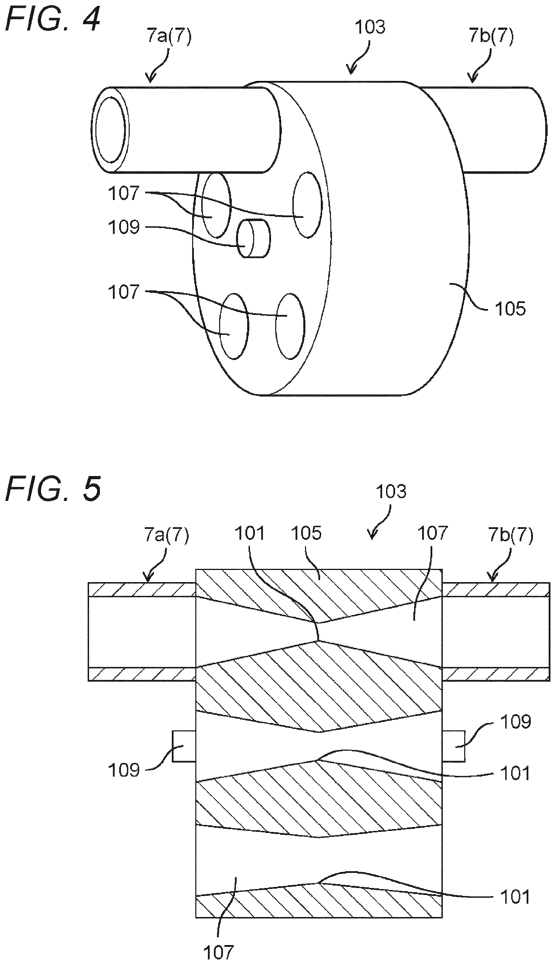

A second exemplary embodiment will be described with reference to Figs. 4 and 5. Fig. 4 is an enlarged perspective view of a main part of nozzle 7 of oral cavity washing device 1 according to the second exemplary embodiment. Fig. 5 is an enlarged sectional view of the main part of nozzle 7 of oral cavity washing device 1 according to the second exemplary embodiment.

In oral cavity washing device 1 according to the present exemplary embodiment, narrow portion 101 includes selector 103 formed with a plurality of different sectional areas.

The same configurations as those of the first exemplary embodiment will be denoted by the same symbols, and the description of the configurations and functions will be omitted by referring to the first exemplary embodiment, but effects obtained from the same configurations as those of the first exemplary embodiment are the same.

As illustrated in Figs. 4 and 5, nozzle 7 is divided into a portion on the suction side and a portion on the discharge side of the washing liquid. The portion on the suction side is denoted by reference numeral "7a". The portion on the discharge side is denoted by reference numeral "7b". Hereinafter, the portion on the suction side and the portion on the discharge side of the washing liquid denoted by reference numerals "7a" and "7b", respectively, are referred to as divided nozzles 7a, 7b. Narrow portion 101 is disposed between divided nozzles 7a, 7b. Narrow portion 101 includes selector 103. Selector 103 includes base 105 and a plurality of communication holes 107.

Base 105 is formed in an annular shape and is disposed between divided nozzles 7a, 7b. At the center of base 105, shaft 109 integrally formed with base 105 is formed so as to protrude toward nozzles 7a, 7b. That is, shaft 109 protrudes in the dividing direction of nozzle 7. In other words, shaft 109 protrudes in the left-right directions in Figs. 4 and 5. Shaft 109 is pivotally supported by a support (not illustrated) formed integrally with divided nozzles 7a, 7b. Thus, base 105 is rotatably disposed between divided nozzles 7a, 7b. A seal member (not illustrated) seals between base 105 and divided nozzles 7a, 7b.

The plurality of communication holes 107 penetrate base 105 in an axial direction. The sectional areas of the openings on both sides of each communication hole 107 are set to be equal to the sectional areas of divided nozzles 7a, 7b. Thus, when base 105 rotates and one communication hole 107 selected from the plurality of communication holes 107 is positioned between divided nozzles 7a, 7b, divided nozzles 7a, 7b communicate with each other via one communication hole 107 to cause the washing liquid to circulate. The plurality of (here, six) communication holes 107 are disposed at equal intervals in the circumferential direction of base 105.

Narrow portion 101 formed with a sectional area smaller than the sectional areas of the openings on both sides is provided at a central portion of each communication hole 107. Narrow portions 101 of the plurality of communication holes 107 have different sectional areas from each other. Thus, rotating base 105 and disposing one desired communication hole 107 between divided nozzles 7a, 7b makes it possible to obtain desired performance such as performance specialized for removing dental plaques, for example.

In this manner, in oral cavity washing device 1 according to the second exemplary embodiment, narrow portion 101 includes selector 103 formed with a plurality of different sectional areas. Thus, selecting a desired sectional area at selector 103 makes it possible to obtain a desired liquid flow of washing liquid with one nozzle 7.

### (Third exemplary embodiment)

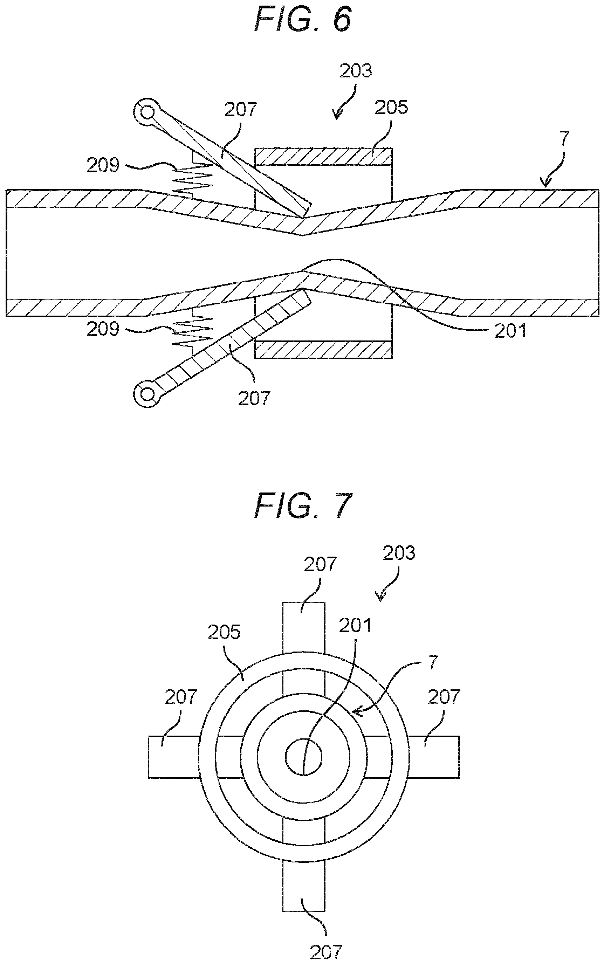

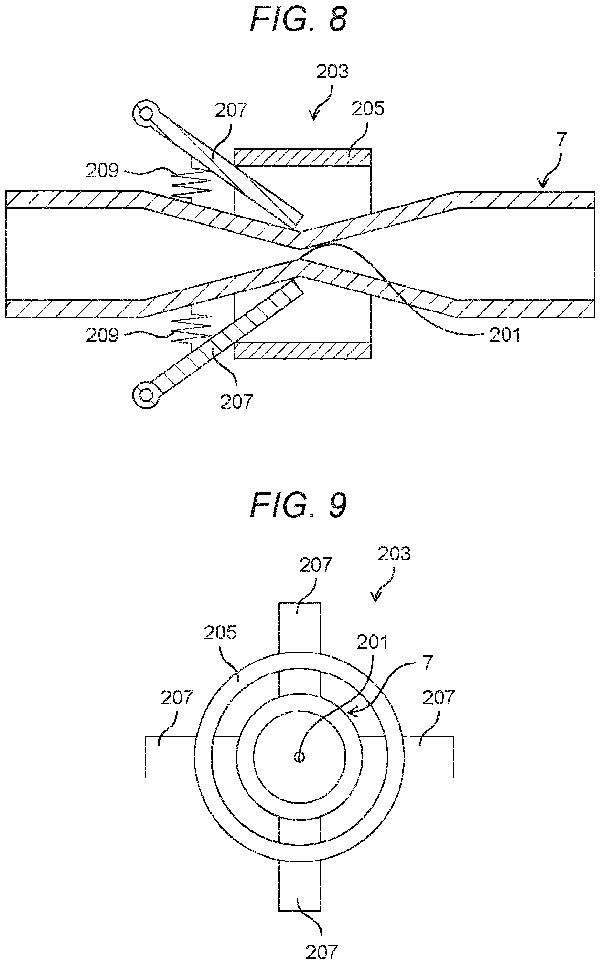

A third exemplary embodiment will be described with reference to Figs. 6 to 9. Fig. 6 is an enlarged sectional view of a main part of nozzle 7 of oral cavity washing device 1 according to the third exemplary embodiment. Fig. 7 is a front view of Fig. 6. Fig. 8 is an enlarged sectional view of the main part of nozzle 7 of oral cavity washing device 1 according to the third exemplary embodiment. Fig. 9 is a front view of Fig. 8.

In oral cavity washing device 1 according to the present exemplary embodiment, narrow portion 201 includes variable portion 203 that changes a sectional area from the outside of nozzle 7.

The same configurations as those of the first exemplary embodiment will be denoted by the same symbols, and the description of the configurations and functions will be omitted by referring to the first exemplary embodiment, but effects obtained from the same configurations as those of the first exemplary embodiment are the same.

As illustrated in Figs. 6 to 9, the suction side and discharge side of nozzle 7 for the washing liquid are formed of a hard material, for example, resin, metal, or the like. A portion located between the suction side and the discharge side of nozzle 7 is formed of a soft material, for example, resin, metal, or the like. Narrow portion 201 is disposed in a portion formed of a soft material. Narrow portion 201 includes variable portion 203. Variable portion 203 includes tubular member 205 and at least one pressing member 207. At least one pressing member 207 may include two or more pressing members 207.

Nozzle 7 is inserted into tubular member 205. Tubular member 205 is supported in a radial direction via a support (not illustrated) integrally provided on the outer periphery of nozzle 7. Tubular member 205 is disposed so as to be movable in a length direction of nozzle 7 on the outer periphery of nozzle 7 via the support.

Pressing member 207 is formed in a plate shape. One end in a length direction of pressing member 207 is pivotally supported via a pivot support (not illustrated) integrally provided on the outer periphery of nozzle 7. Thus, the other end in the length direction of pressing member 207 moves in a direction away from the outer periphery of nozzle 7 and in a direction approaching the outer periphery of nozzle 7. More specifically, the other end in the length direction of pressing member 207 moves in one of a direction away from the outer periphery of nozzle 7 and a direction approaching the outer periphery of nozzle 7.

One end in the length direction of pressing member 207 is disposed at a position farther from the outer periphery of nozzle 7 than the other end in the length direction. Bias member 209 that biases pressing member 207 in a direction away from the outer periphery of nozzle 7 is disposed between one end side in the length direction of pressing member 207 and the outer periphery of nozzle 7. The other end in the length direction of pressing member 207 is disposed inside tubular member 205 and abuts on a portion of nozzle 7 formed of a soft material. The plurality of (here, four) pressing members 207 are disposed at equal intervals in the circumferential direction with respect to the outer periphery of nozzle 7.

As illustrated in Figs. 6 and 7, in such variable portion 203, pressing member 207 presses the outer periphery of nozzle 7 when tubular member 205 abuts on pressing member 207. Narrow portion 201 having a smaller sectional area than other portions is formed in nozzle 7 by pressing with pressing member 207. As illustrated in Figs. 8 and 9, when tubular member 205 moves toward pressing member 207, the abutment with pressing member 207 is strengthened, and pressing member 207 further presses the outer periphery of nozzle 7. Further pressing of pressing member 207 reduces the sectional area of narrow portion 201.

In this manner, the sectional area of narrow portion 201 can be changed from the outside of nozzle 7 via pressing member 207 by the movement of tubular member 205. Thus, adjusting the position of tubular member 205 makes it possible to adjust the sectional area of narrow portion 201 in accordance with the liquid flow of the washing liquid. To increase the reduced sectional area of narrow portion 201, tubular member 205 may be moved, and for example, a jig may be inserted into nozzle 7.

When the portion to be deformed by variable portion 203 of nozzle 7 is formed of a plastically deformable material, the sectional area of narrow portion 201 can be maintained, and thus variable portion 203 can be configured to be detachable from nozzle 7. The portion of nozzle 7 to be deformed by variable portion 203 may be formed of a restorable material such as an elastic body. In this case, the reduced sectional area of narrow portion 201 can be increased by the movement of tubular member 205.

As just described, in oral cavity washing device 1 according to the third exemplary embodiment, narrow portion 201 includes variable portion 203 that changes a sectional area from the outside of nozzle 7. Thus, adjusting variable portion 203 makes it possible to adjust the sectional area of narrow portion 201 with one nozzle 7 according to the liquid flow of the washing liquid.

The exemplary embodiment described above is to exemplify the techniques in the present disclosure, and thus, various modifications, replacements, additions, omissions, and the like can be made in the scope of claims or in an equivalent scope of the claims.

In the first to third exemplary embodiments, the sectional shape of the inside of nozzle 7 is circular. The term "circular" includes "elliptical". The sectional shape of the inside of nozzle 7 is not limited to a circular shape. As an example, the sectional shape of the inside of nozzle 7 may be a polygonal shape such as a triangle, a quadrangle, or a pentagon. In other words, although the sectional shape of the inner surface of nozzle 7 is circular in the first to third embodiments, the sectional shape of the inner surface of nozzle 7 may be a non-circular shape. That is, when the sectional shape is circular, the sectional area is changed by changing the inner diameter of nozzle 7. When the sectional shape is elliptical, the sectional area can be changed by changing at least one of the major axis (that is, the long axis) and the minor axis (that is, the short axis) of the ellipse. For example, when the sectional shape is a polygon such as a quadrangle, the sectional area can be changed by deforming at least one of the plurality of wall surfaces constituting the inner surface so as to change the distance between the one surface and another surface (for example, a surface facing the one surface).

### INDUSTRIAL APPLICABILITY

The present disclosure is applicable to an oral cavity washing device that discharges liquid from a nozzle.

### REFERENCE MARKS IN THE DRAWINGS

- 1: oral cavity washing device
- 3: tank
- 5: device body
- 7: nozzle
- 7a: divided nozzle
- 7b: divided nozzle
- 9: suction path
- 11: pump
- 13: motor
- 15: cam
- 17: piston
- 19: pump chamber
- 21: discharge path
- 23: narrow portion
- 101: narrow portion
- 103: selector
- 105: base
- 107: communication hole
- 109: shaft
- 201: narrow portion
- 203: variable portion
- 205: tubular member
- 207: pressing member
- 209: bias member

## Claims

1. An oral cavity washing device comprising:
a device body comprising a pump that discharges a washing liquid; and
a nozzle that is provided in the device body and discharges the washing liquid discharged by the pump to an outside,
wherein
the nozzle comprises a narrow portion in such a manner that the sectional area thereof is set to be smaller than that of another portion of the nozzle, and that the sectional area thereof is variable.

2. The oral cavity washing device according to claim 1, wherein the sectional area of the narrow portion is changed by using an external force.

3. An oral cavity washing device comprising:
a device body comprising a pump that discharges a washing liquid; and
a nozzle that is provided in the device body and discharges the washing liquid discharged by the pump to an outside,
wherein
the nozzle is divided into two divided nozzles,
a selector is disposed between the two divided nozzles,
the selector comprises a plurality of communication holes,
sectional areas of the plurality of communication holes are different from each other, and
the selector is configured in such a manner that one communication hole selected from the plurality of communication holes communicates with the two divided nozzles.

4. The oral cavity washing device according to claim 1 or 2, wherein
the narrow portion comprises a variable portion that varies the sectional area from outside the nozzle.
